# FASCICULE DE BREVET EUROPEEN

(11) **EP 1 509 625 B1**
(45) Date de publication et mention de la délivrance du brevet: **22.10.2008**
(21) Numéro de dépôt: 03757099.1
(22) Date de dépôt: 03.06.2003
(51) Int. Cl.: C12Q 1/68, G01N 33/543

(54) **METHODE POUR DETECTION DES REACTIONS D'HYBRIDATION ET D'IMMUNOLOGIQUES**
VERFAHREN ZUM SIMULTANEN NACHWEIS VON HYBRIDISIERUNGS UND IMMUNOLOGISCHEN REAKTIONEN
PROCEDE DE DETECTION SIMULTANEE DE REACTIONS D'HYBRIDATION ET IMMUNOLOGIQUES

(30) Priorité: 05.06.2002 FR 0206883
(43) Date de publication de la demande: 02.03.2005
(73) Titulaire: BIOMERIEUX, 69280 Marcy l'Etoile (FR)
(72) Inventeur: MANDRAND, Bernard, F-69100 Villeurbanne (FR); PERRIN, Agnès, F-69007 LYON (FR); THERETZ, Alain, F-69130 Ecully (FR)
(74) Mandataire: Bitaud, Valérie Marie-Odile
(86) Numéro de dépôt international: PCT/FR2003/001659
(87) Numéro de publication internationale: WO 2003/104490

(56) Documents cités:
- WO-A-00/75644
- WO-A-01/61040
- WO-A-01/88511
- WO-A-93/23565
- WO-A-97/29206
- US-A- 5 837 465
- US-A- 5 849 545
- MOLE L ET AL: "Stabilities of quantitative plasma culture for human immunodeficiency virus, RNA, and p24 antigen from samples collected in VACUTAINER CPT and standard VACUTAINER tubes" JOURNAL OF CLINICAL MICROBIOLOGY 1994 UNITED STATES, vol. 32, no. 9, 1994, pages 2212-2215, XP008017314 ISSN: 0095-1137
- DATABASE BIOSIS [Online] BIOSCIENCES INFORMATION SERVICE, PHILADELPHIA, PA, US; 1998, WU NANPING ET AL: "Study on the correlations of P24 antigen, HIV DNA, HIV RNA in 139 HIV infected cases." XP002241359 Database accession no. PREV199800512148 & ZHONGHUA WEISHENGWUXUE HE MIANYIXUE ZAZHI, vol. 18, no. 5, 1998, pages 359-362, ISSN: 0254-5101
- DATABASE BIOSIS [Online] BIOSCIENCES INFORMATION SERVICE, PHILADELPHIA, PA, US; 15 janvier 1999 (1999-01-15), KORALNIK I J ET AL: "JC virus DNA load in patients with and without progressive multifocal leukoencephalopathy." XP002241360 Database accession no. PREV199900128080 & NEUROLOGY, vol. 52, no. 2, 15 janvier 1999 (1999-01-15), pages 253-260, ISSN: 0028-3878

## Description

La présente invention concerne un nouveau procédé de détection simultanée de réactions d'hybridation et immunologiques, ainsi que son utilisation dans le diagnostic thérapeutique et industriel, ainsi que dans l'identification et/ou la quantification de molécules biologiques.

Le diagnostic ou le suivi de pathologies nécessitent le plus souvent des détections d'hybridation et/ou des détections immunologiques. Ainsi, dans le cas du diagnostic du SIDA, il peut être nécessaire de rechercher à la fois la présence de la protéine p24, de l'anticorps anti-protéine d'enveloppe virale et de l'ARN viral, ou d'anticorps anti-protéine p24 et de l'ARN viral.

La détection simultanée des réactions d'hybridation et immunologiques dans les mêmes conditions opératoires et dans le même milieu de réaction permettrait de faciliter le diagnostic des pathologies requérant une telle détection double.

La détection de réactions immunologiques et d'hybridation utilisant des paramètres communs a déjà été étudiée dans l'art antérieur.

Ainsi, la demande de brevet WO01/86296 décrit un procédé conçu pour la détection simultanée d'un nombre important d'analytes à l'aide d'un panel de marqueurs spécifiques de chaque analyte. Toutefois, ce procédé ne décrit pas la détection simultanée à la fois de réactions d'hybridation et immunologiques et utilise des conditions opératoires de révélation différentes pour chaque marqueur spécifique de chaque analyte.

La demande de brevet WO01/61040 décrit également un procédé de détection simultanée d'analytes à l'aide de nanocristaux semi-conducteurs comme marqueurs de détection. La lecture des réactions d'hybridation ou immunologiques est réalisée grâce à la variation de la longueur d'onde de lecture. Toutefois, ce procédé a pour inconvénient que l'échantillon à analyser doit être découpé en plusieurs échantillons de sorte que chaque analyte à étudier est soumis à des conditions opératoires différentes.

La demande de brevet WO97/29206 décrit un procédé de dosage d'analytes pour identifier l'origine d'un échantillon biologique, en utilisant un récipient de réaction ayant une pluralité de cellules de réaction, un premier ensemble de cellules de réaction contenant au moins un réactif pour obtenir un profil anticorps et un deuxième ensemble de cellules de réaction contenant au moins un réactif pour obtenir un profil diagnostic.

Le brevet US5,837,465 décrit l'utilisation de luciférase conjuguée à un anticorps ou un acide nucléique à titre de marqueur. Les conjugués ainsi obtenus sont utilisés dans des tests diagnostiques tels des immunodosages. Ce brevet suggère que les conjugués à base d'acide nucléique peuvent être utilisés dans des réactions d'hybridation. Il suggère également le dosage simultané d'antigènes et d'acides nucléiques en utilisant des guides d'onde ou zones de puce différents sans précisions quant aux conditions de réaction.

Les procédés de l'art antérieur ont donc pour inconvénients que la détection simultanée de réactions d'hybridation et immunologiques ne peut pas avoir lieu dans les mêmes conditions opératoires et dans le même milieu de réaction, de sorte que la détection simultanée est complexe et coûteuse.

La demanderesse a maintenant mis au point un nouveau procédé de détection simultanée de réactions d'hybridation et immunologiques palliant les inconvénients ci-dessus.

Ainsi, l'invention a pour objet un procédé de détection simultanée de réactions d'hybridation et immunologiques dans un échantillon susceptible de contenir des analytes cibles constitués d'au moins un acide nucléique et d'au moins un autre ligand de nature différente, comprenant les étapes consistant à :
(i) déposer une quantité connue en volume de l'échantillon dilué dans un tampon de réaction, sur une surface de capture préalablement revêtue des partenaires de capture desdits analytes cibles, lesdits partenaires de capture consistant en au moins une sonde nucléique et au moins un antiligand,
(ii) mettre à réagir à une température comprise entre 15°C et 60°C et
(iii) visualiser les réactions d'hybridation et immunologiques ainsi obtenues, caractérisé en ce que le tampon de réaction a une force ionique comprise entre 0,4 et 1 M, a un pH compris entre 7 et 8 et contient un agent tensioactif, ainsi que l'utilisation de ce procédé pour la détection de maladies d'origine infectieuse, par exemple virale, et métaboliques, pour le diagnostic industriel de la présence de bactéries, ainsi que pour l'identification et/ou la quantification de molécules biologiques.

Un autre objet de l'invention consiste en des kits selon les revendications 19-22 d'analyse biologique ou diagnostique utiles pour la mise en oeuvre du procédé de l'invention.

Le procédé de l'invention est un procédé simple à mettre en oeuvre qui permet, contre toute attente, de détecter dans un échantillon, dans les mêmes conditions opératoires que sont le milieu et la température de réaction, la présence d'analytes cible constitués d'au moins un acide nucléique et d'au moins un autre ligand de nature différente. La présence desdits analytes cible est mise en évidence par la visualisation de réactions d'hybridation et de réactions immunologiques.

On entend par réaction d'hybridation toute réaction entre un acide nucléique de capture et un acide nucléique cible, et on entend par réaction immunologique toute réaction entre un antiligand de capture et un ligand cible de nature autre que nucléique.

On entend par acide nucléique les oligonucléotides, les acides désoxyribonucléiques et les acides ribonucléiques, ainsi que leurs dérivés.

Le terme « oligonucléotide » désigne un enchaînement d'au moins 2 nucléotides (désoxyribonucléotides ou ribonucléotides, ou les deux), naturels ou modifiés, susceptibles de s'hybrider, dans des conditions appropriées d'hybridation, avec un oligonucléotide au moins partiellement complémentaire. Par nucléotide modifié, on entend par exemple un nucléotide comportant une base modifiée et/ou comportant une modification au niveau de la liaison intemucléotidique et/ou au niveau du squelette. A titre d'exemple de base modifiée, on peut citer l'inosine, la méthyl-5-désoxycytidine, la diméthylamino-5-désoxyuridine, la diamino-2,6-purine et la bromo-5-désoxyuridine. Pour illustrer une liaison internucléotidique modifiée, on peut mentionner les liaisons phosphorothioate, N-alkylphosphoramidate, alkylphosphonate et alkylphosphodiester. Les alpha-oligonucléotides tels que ceux décrits dans FR-A-2 607 507, les LNA tels que phosphorothioate-LNA and 2'-thio-LNA décrits dans Bioorganic & Médicinal Chemistry Letters, Volume 8, Issue 16, 18 August 1998 ,pages 2219-2222, et les PNA qui font l'objet de l'article de M. Egholm et al., J. Am. Chem. Soc. (1992), 114, 1895-1897, sont des exemples d'oligonucléotides constitués de nucléotides dont le squelette est modifié.

On entend par ligand de nature différente, autre que nucléique, toute molécule différente d'un acide nucléique, capable de liaison avec un partenaire de liaison spécifique. Ce partenaire de liaison est appelé antiligand lorsqu'il consiste en le partenaire de capture. Pour des raisons de commodité, on utilisera ci-après les termes ligand et antiligand pour désigner tout composé capable d'une réaction immunologique, mais pas d'une réaction d'hybridation.

A titre de ligand, on peut citer par exemple les antigènes, les anticorps, les polypeptides, les protéines, les haptènes, les sucres, les enzymes et leurs substrats.

A titre d'antiligand, on peut citer les mêmes exemples que ceux concernant les ligands, ainsi que les lectines, les récepteurs cellulaires et les aptamères.

Le terme « antigène » désigne un composé susceptible d'être reconnu par un anticorps dont il a induit la synthèse par une réponse immune.

Le terme « anticorps » inclut les anticorps polyclonaux ou monoclonaux, les anticorps obtenus par recombinaison génétiques et des fragments d'anticorps.

Les anticorps polyclonaux peuvent être obtenus par immunisation d'un animal avec au moins un antigène cible d'intérêt, suivie de la récupération des anticorps recherchés sous forme purifiée, par prélèvement du sérum dudit animal, et séparation desdits anticorps des autres constituants du sérum, notamment par chromatographie d'affinité sur une colonne sur laquelle est fixée un antigène spécifiquement reconnu par les anticorps, notamment un antigène cible d'intérêt.

Les anticorps monoclonaux peuvent être obtenus par la technique des hybridomes dont le principe général est rappelé ci-après.

Dans un premier temps, on immunise un animal, généralement une souris, (ou des cellules en culture dans le cadre d'immunisations *in vitro*) avec un antigène cible d'intérêt, dont les lymphocytes B sont alors capables de produire des anticorps contre ledit antigène. Ces lymphocytes producteurs d'anticorps sont ensuite fusionnés avec des cellules myélomateuses "immortelles" (murines dans l'exemple) pour donner lieu à des hybridomes. A partir du mélange hétérogène des cellules ainsi obtenu, on effectue alors une sélection des cellules capables de produire un anticorps particulier et de se multiplier indéfiniment. Chaque hybridome est multiplié sous la forme de clone, chacun conduisant à la production d'un anticorps monoclonal dont les propriétés de reconnaissance vis-à-vis de l'antigène tumoral d'intérêt pourront être testées par exemple en ELISA, par immunotransfert en une ou deux dimensions, en immunofluorescence, ou à l'aide d'un biocapteur. Les anticorps monoclonaux ainsi sélectionnés, sont par la suite purifiés notamment selon la technique de chromatographie d'affinité décrite ci-dessus.

Les fragments d'anticorps sont tels qu'ils conservent la fonction de liaison avec leur partenaire de liaison ou capture.

Par « polypeptide », on entends un enchaînement d'au moins deux acides aminés. Par acides aminés, on entend les acides aminés primaires qui codent pour les protéines, les acides aminés dérivés après action enzymatique comme la *trans*-4-hydroxyproline et les acides aminés naturels, mais non présents dans les protéines comme la norvaline, la N-méthyl-L-leucine, la staline (Hunt S. dans Chemistry and Biochemistry of the amino acids, Barett GC, ed., Chapman and Hall, London, 1985), les acides aminés protégés par des fonctions chimiques utilisables en synthèse sur support solide ou en phase liquide et les acides aminés non naturels.

Le terme « protéine » inclut les holoprotéines et les hétéroprotéines comme les nucléoprotéines, les lipoprotéines, les phosphoprotéines, les métalloprotéines et les glycoprotéines aussi bien fibreuses que globulaires.

Le « terme » haptène désigne des composés non immunogènes, c'est-à-dire incapables par eux-mêmes de promouvoir une réaction immunitaire par production d'anticorps, mais capables d'êtres reconnus par des anticorps obtenus par immunisation d'animaux dans des conditions connues, en particulier par immunisation avec un conjugué haptène-protéine. Ces composés ont généralement une masse moléculaire inférieure à 3000 Da, et le plus souvent inférieure à 2000 Da, et peuvent être par exemple des peptides glycosylés, des métabolites, des vitamines, des hormones, des prostaglandines, des toxines ou divers médicaments, les nucléosides et les nucléotides.

Les enzymes et leurs substrats sont bien connus de l'homme du métier. L'utilisation d'une enzyme à titre d'antiligand de capture permet de rechercher dans l'échantillon testé les analogues de substrat de l'enzyme.

Les lectines sont capables de reconnaître les sucres par un mécanisme bien connu de l'homme du métier, comme décrit dans Biochemistry, 4th Edition, G.L. Zubay, 1998, The Mc Graw-Hill Companies, USA, Boston.

Les récepteurs utilisés à titres d'antiligand sont des protéines de surface capables de se lier avec le ligand cible protéine d'enveloppe. Des exemples de tels récepteurs comprennent le récepteur chemokine CXCR4 de la protéine d'enveloppe HIV gp120 (Corananiti, M.T., 2001, Neuroscience Letters, 312(2), 67-70).

Les aptamères sont des partenaires de captures de nature protéique et nucléique qui ont pour fonction d'agir en tant qu'anticorps et de se lier à des ligands protéiques (Toulmé, J.J. et Giege, R. , 1998, Medecine Science, 14(2), 155-166).

L'échantillon testé dans le procédé de l'invention peut être soit biologique, soit industriel.

A titre d'échantillon biologique, on peut citer tout fluide biologique susceptible de contenir des analytes cible tel que le sang, la lymphe, le liquide céphalo-rachidien, le prélèvement de gorge, les frottis vaginaux et les urines.

A titre d'échantillon industriel, on peut citer tout échantillon provenant de l'industrie pour lequel une analyse biologique est nécessaire. Les échantillons provenant de l'industrie alimentaire (plats préparés, eaux traitées) constituent un exemple d'échantillon industriel.

Le tampon de réaction dans lequel est dilué l'échantillon est un tampon de réaction intermédiaire entre un tampon d'hybridation et un tampon utilisé dans les réactions immunologiques, le tampon de réaction a une force ionique comprise entre 0,4 et 1M, a un pH compris entre 7 et 8 et contient un agent tensioactif.

A titre de tampon de réaction, on peut citer les tampons à base de sels de phosphate, de sels de sodium, de sels de lithium et l'HEPES et à titre d'agent tensioactif, on peut citer Tween 20, Tween 80 et le Triton.

L'originalité de l'invention consiste donc en ce qu'on peut détecter à la fois des réactions d'hybridation et des réactions immunologiques dans ce tampon de réaction.

La température de réaction du procédé de l'invention est comprise entre 15 et 60°C. A une température inférieure à 15°C, les réactions d'hybridation risquent d'être peu spécifiques, notamment si les oligonucléotides ont une taille supérieure à 8 pdb et à une température supérieure à 60°C, on peut rencontrer des problèmes au niveau des ligands et antiligands. En effet, à ces températures, certaines protéines sont dénaturées et les réactions d'hybridation ne sont possibles qu'avec des oligonucléotides de grandes tailles, supérieures à 30 pdb.

Selon un mode de réalisation, la température de réaction est comprise entre 35 et 45°C, de préférence entre 37 et 41°C, les températures de 37 et 41 °C étant davantage préférées.

La surface de capture sur laquelle on dépose l'échantillon à tester peut être toute surface sur laquelle on peut fixer des sondes nucléiques et des antiligands. A titre de surface de capture, on peut citer les micropuits, les microplaques, les surfaces polymères, les membranes, les lames de miscroscopie, les supports inorganiques fonctionnalisés ou non, tels que silice, verre, mica ou quartz, les surfaces métalliques telles que d'or et d'argent et les particules et microparticules, notamment magnétiques.

Pour le procédé de l'invention, la surface de capture est préalablement revêtue des partenaires de capture desdits analytes cible, à savoir des sondes nucléiques et des antiligands.

Le dépôt des partenaires de capture peut être effectué selon le procédé décrit dans la demande de brevet de la Demanderesse FR00/14691 (FR2 816 711) qui est un procédé de dépôt sans contact particulièrement adapté au format de microplaque 96 puits. Ce procédé consiste à éjecter des nanogouttes calibrées à travers une buse, sous l'effet d'un choc mécanique, sur le fond des puits de la microplaque. On obtient ainsi à chaque choc une goutte dont le diamètre varie selon celui de la buse, de 50 à 500 µm environ.

Le dépôt de ces partenaires peut être également effectué manuellement à l'aide de capillaires de verre dont le diamètre interne avoisine la centaine de microns. Dans ce cas, le capillaire doit entrer en contact avec la surface de dépôt pour que le dépôt ait lieu, ce qui entraîne un léger choc avec la surface de dépôt.

La visualisation des réactions d'hybridation et immunologiques peut être effectuée par tout moyen de détection, tels que des moyens directs ou indirects.

Dans le cas de la détection directe, c'est-à-dire sans l'intermédiaire d'un marquage, on observe les réactions d'hybridation et immunologiques par exemple par résonance plasmon ou par voltamétrie cyclique sur une électrode portant un polymère conducteur.

Dans le cas de la détection indirecte, c'est-à-dire par l'intermédiaire d'un marquage, le marquage peut être effectué soit directement sur les analytes cible, soit par l'intermédiaire d'un partenaire de liaison spécifique desdits analytes cible préalablement marqué.

On entend par partenaire de liaison spécifique des analytes cible tout partenaire capable de se lier avec l'analyte cible et on donnera à titre d'exemples les acides nucléiques, les antigènes, les anticorps, les fractions d'anticorps, les protéines, les haptènes, les oligonucléotides ou polynucléotides et les substrats enzymatiques.

Selon un mode de réalisation dans lequel la visualisation des réactions d'hybridation et immunologiques est effectuée par l'intermédiaire de partenaires de liaison spécifique desdits analytes cible préalablement marqués, le procédé de l'invention comprend l'étape supplémentaire suivante (i'), placée entre l'étape (i) et l'étape (ii), consistant à :
(i') ajouter des partenaires de liaison spécifiques desdits analytes cible, lesquels partenaires ont été préalablement conjugués à des marqueurs.

Selon un autre mode de réalisation dans lequel la visualisation des réactions d'hybridation et immunologiques est également effectuée par l'intermédiaire de partenaires de liaison spécifique desdits analytes cible préalablement marqués, le procédé de l'invention comprend les deux étapes supplémentaires suivantes (ii') et (ii"), placées entre l'étape (ii) et l'étape (iii), consistant à :
(ii') ajouter des partenaires de liaison spécifiques desdits analytes cible, lesquels partenaires ont été préalablement conjugués à des marqueurs, et
(ii") mettre à réagir à une température comprise entre 15°C et 60°C.

Ainsi, dans ce cas, le procédé de l'invention comprend deux étapes de réaction, l'une pour la liaison entre les partenaires de capture des analytes cibles et les analytes cibles (étape (ii)), et l'autre entre le conjugué partenaire de capture/analyte cible et le partenaire de liaison spécifique de l'analyte cible (étape (ii")).

Selon encore un autre mode de réalisation, la visualisation des réactions d'hybridation est effectuée grâce au marquage préalable des analytes cible du type acide nucléique et la visualisation des réactions immunologiques est effectuée grâce au marquage des partenaires de liaison spécifique desdits analytes cible du type ligand. En conséquence, dans ce mode de réalisation, comme les acides nucléiques cibles ont été préalablement marqués, les seuls partenaires de liaison à ajouter dans l'étape (i') ou (ii') sont le ou les partenaire(s) spécifique(s) du ligand, le(s)quel(s) a(ont) été préalablement marqué(s).

Les réactions immunologiques peuvent également être détectées selon une méthode dite de compétition. On ajoute alors dans le milieu de réaction (étapes (i') et (ii')), à la place du ou des partenaires spécifiques de ligand, le ou les ligands susceptibles d'être contenus dans l'échantillon biologique, lesquels ont été préalablement marqués, ce qui constitue un autre mode de réalisation de l'invention. Dans ce cas, le signal de détection est maximal en l'absence du ligand recherché, puis diminue progressivement à mesure que la concentration en ligand recherché, non marqué, augmente par la réaction de compétition.

La surface de capture peut être rincée après les étapes de réaction (étapes (ii) et/ou (ii")) de chaque mode de réalisation de l'invention, pour éliminer de la surface de capture les molécules qui n'ont pas réagi et les molécules faiblement adsorbées, sans interaction spécifique. Ainsi, la visualisation des réactions d'hybridation et immunologiques s'en trouve améliorée. Ceci constitue un autre mode de réalisation de l'invention.

Comme milieu de rinçage, on peut utiliser par exemple du PBS-tween , comme décrit par Perrin A., et al, dans J. Immunological Methods, 1999, 224, 77-87.

Par marquage, on entend la fixation d'un marqueur capable de générer directement ou indirectement un signal détectable. Une liste non limitative de ces marqueurs consiste en :
- les enzymes qui produisent un signal détectable par exemple par colorimétrie, fluorescence, luminescence, comme la péroxydase de raifort, la phosphatase alcaline, la α-galactosidase, la glucose-6-phosphate déshydrogénase,
- les chromophores comme les composés, luminescents, colorants,
- les molécules radioactives comme le ³²P, le ³⁵S ou le ¹²⁵I,
- les molécules fluorescentes telles que la fluorescéine, la rhodomine, l'alexa ou les phycocyanines, et
- les particules telles que les particules en or, en latex magnétique, les liposomes.

Des systèmes indirects peuvent être aussi utilisés, comme par exemple par l'intermédiaire d'un autre couple ligand/antiligand. Les couples ligand/antiligand sont bien connus de l'homme du métier, et on peut citer par exemple les couples suivants : biotine/streptavidine, haptène/anticorps, antigène/anticorps, peptide/anticorps, sucre/lectine, polynucléotide/complémentaire du polynucléotide. Dans ce cas, c'est le ligand qui porte l'agent de liaison. L'antiligand peut être détectable directement par les marqueurs décrits au paragraphe précédent ou être lui-même détectable par un ligand/anti-ligand.

Ces systèmes de détection indirects peuvent conduire, dans certaines conditions, à une amplification du signal. Cette technique d'amplification du signal est bien connue de l'homme du métier, et l'on pourra se reporter aux demandes de brevet antérieures FR98/10084 ou WO-A-95/08000 de la demanderesse ou à l'article J. Histochem. Cytochem. 45 : 481-491, 1997.

Le marquage préalable des analytes cible du type acide nucléique peut être effectué par incorporation directe ou indirecte de marqueur par une polymérase.

Le marquage des partenaires de liaison spécifiques des analytes cibles est largement connu de l'homme du métier et est décrit par exemple par Greg T. Hermanson dans Bioconjugate Techniques, 1996, Academic Press Inc, 525B Street, San Diego, CA92101 USA.

Selon le type de marquage du conjugué utilisé, comme par exemple en utilisant une enzyme, l'homme du métier ajoutera des réactifs permettant la visualisation du marquage.

Ainsi, selon un mode de réalisation du procédé de l'invention, on ajoute le ou les substrat(s) spécifiques du ou des marqueur(s) avant l'étape (iii) de visualisation des réactions d'hybridation et immunologiques.

De tels réactifs sont largement connus de l'homme du métier et sont décrits notamment dans Principles and Practice of Immunoessay, 2nd Edition, Edited by C. Price, D.J. Newman Stockton Press, 1997, 345 Park Avenue South, New York.

Les éléments marqués aux fins du procédé de l'invention, à savoir les partenaires de liaison spécifiques d'acide nucléique, les partenaires de liaison spécifiques de ligand, les acides nucléiques cibles contenus dans l'échantillon biologique et les ligands susceptibles d'être contenus dans l'échantillon biologique, peuvent être marqués avec des marqueurs différents ou avec le même marqueur, la deuxième solution étant préférée.

Les réactions d'hybridation et immunologiques détectées par le procédé de l'invention peuvent être représentatives de la présence d'une maladie unique. Dans ce cas, les partenaires de capture appliqués sur la surface de capture sont des marqueurs de la même maladie, ce qui constitue un mode de réalisation de l'invention.

Ainsi par exemple, dans le cas du diagnostic précoce du SIDA, on peut rechercher à la fois la présence de l'anticorps anti-protéine p24 et de l'ARN viral. Les partenaires de capture présents à la surface de capture peuvent donc être la protéine p24, et une sonde nucléique capable d'une réaction d'hybridation avec l'ARN viral.

Les réactions d'hybridation et immunologiques détectées par le procédé de l'invention peuvent également être représentatives de différentes maladies. Dans ce cas, les partenaires de capture sont des marqueurs de maladies différentes, ce qui constitue un autre mode de réalisation de l'invention. Ainsi, le procédé de l'invention peut être utilisé pour le criblage, à partir d'un seul échantillon biologique, dans les mêmes conditions opératoires et en même temps, de plusieurs maladies différentes mises en évidence soit à partir de la détection d'acide nucléique, soit à partir de la détection de ligand d'une autre nature. Ce procédé peut par exemple être utile dans l'analyse de sang issu d'un don, avant transfusion, dans lequel on veut vérifier qu'il n'y a pas présence de pathogènes.

Les maladies qui peuvent être détectées par le procédé de l'invention sont toutes maladies dont on connaît au moins un couple analyte cible et partenaire de capture capables d'inter-réaction.

Ces maladies peuvent être d'origine infectieuse, à savoir virale ou bactérienne, comme le SIDA, les hépatites, ou bien elles peuvent être métaboliques, comme l'hypertyroïdémie ou le diabète.

Ainsi, un autre objet de l'invention consiste en l'utilisation du procédé de l'invention pour la détection de maladies d'origine infectieuse ou métaboliques.

Encore un autre objet de l'invention consiste en l'utilisation du procédé de l'invention dans le diagnostic industriel de la présence de bactéries. En effet, la mise en évidence de la présence de bactéries dans l'industrie, notamment la listéria ou les salmonelles dans l'industrie alimentaire, peut être effectuée par détection de réactions d'hybridation (détection d'ADN ou ARN bactériens) et immunologiques (détection de protéines bactériennes).

Un autre objet de l'invention consiste également en l'utilisation du procédé de l'invention pour l'identification et/ou la quantification de molécules biologiques. En effet, lorsqu'on utilise de nombreux partenaires de capture différents, mettant en évidence différentes nombreuses molécules biologiques, le procédé de l'invention permet d'identifier quelle(s) molécule(s) est(sont) présente(s) dans l'échantillon. De même, lorsqu'on utilise un grand nombre de partenaires de capture mais de nature différente réduite, le procédé de l'invention permet de quantifier les molécules biologiques présentes dans l'échantillon biologique, ce qui peut conduire à définir des profils types d'une pathologie ou d'un état environnemental.

Un autre objet de l'invention consiste en l'utilisation du procédé de l'invention pour la détection simultanée du transcriptome et du protéome d'une cellule ou d'un organisme. La détection simultanée de l'ensemble de l'ARN (transcriptome) et de l'ensemble des protéines (protéome) d'une cellule ou d'un organisme permet ainsi de mettre en évidence un dysfonctionnement potentiel de surexpression des protéines par rapport aux ARN présents et vice-versa.

Pour mettre en oeuvre le procédé de l'invention, on dispose de kits constitués d'au moins un des éléments suivants :
- une surface de capture,
- des partenaires de capture des analytes cible consistant en au moins une sonde nucléique et au moins un autre antiligand de nature différente,
- un tampon de réaction ayant une force ionique comprise entre 0,4 et 1M, un pH compris entre 7 et 8 et contenant un agent tensioactif,
- éventuellement des partenaires spécifiques des ligands cibles préalablement marqués ou des ligands préalablement marqués susceptibles d'être contenus dans l'échantillon à tester et
- éventuellement des partenaires spécifiques des acides nucléiques cibles.

Ces kits, qu'on peut qualifier d'analyse biologique lorsque l'échantillon testé est industriel, ou d'analyse diagnostique lorsque l'échantillon testé est biologique constituent un autre mode de réalisation de l'invention.

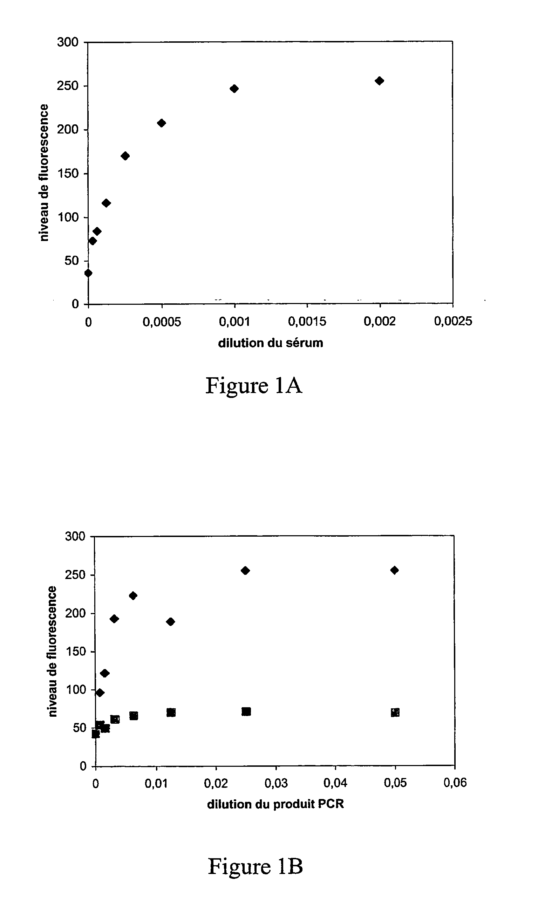

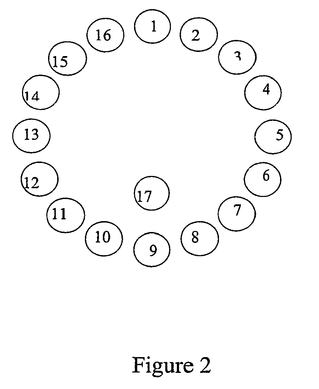

La présente invention sera mieux comprise à l'aide des exemples suivants donnés uniquement à titre illustratif et non limitatif, ainsi qu'à l'aide des figures 1 et 2 annexées, sur lesquelles :
- la figure 1 représente deux graphes montrant d'une part l'évolution de la fluorescence mettant en évidence les réactions immunologiques entre le partenaire de capture protéine p24/polymère AMVE et l'anticorps anti-protéine p24 présent dans des sérums humains, en fonction de la dilution des sérums (figure 1A) et montrant d'autre part l'évolution de la fluorescence représentative des réactions d'hybridation ou d'absence de telles réactions, le cas échéant , entre les partenaires de capture sondes nucléiques C+ (capables de réagir avec la cible ADN du virus VIH) et C- (incapables de réagir avec la cible ADN du virus VIH) et la cible ADN du virus VIH, en fonction de la dilution du produit PCR (figure 1B), et
- la figure 2 représente le plan de dépôt d'une puce de détection multidosage de 17 puits comprenant chacun les partenaires de capture en duplicata, avec :
   - dans les puits 1, 9 et 17 : des partenaires de capture nucléiques pour le virus VIH (C_{HIV}),
   - dans les puits 3 et 11 : des partenaires de capture nucléiques pour le virus de l'hépatite B HBV (C_{HBV}),
   - dans les puits 7 et 15 : des partenaires de capture nucléiques pour le virus de l'hépatite C HCV (C_{HCV}),
   - dans les puits 5 et 13 : des partenaires de capture protéiques pour le virus VIH (protéine d'enveloppe gp160),
   - dans les puits 4 et 12 : des partenaires de capture protéiques pour le virus HCV (protéine core),
   - dans les puits 8 et 16 : des partenaires de capture protéiques pour le virus HBV (2 antigènes de surface différents Ag HBs par puits),et
   - dans les puits 2, 6, 10 et 14 : des partenaires de capture protéiques non spécifiques de cette études, à savoir un anticorps anti-TSH (NSP1, puits 2 et 10) et un anticorps anti-HCG (NSP2, puits 6 et 14).

### Exemple 1 : Préparation des surfaces de capture pour la détection de réactions d'hybridation et immunologigues pour la mise en évidence de la présence du virus VIH chez un patient

### 1.1 Préparation du partenaire de capture susceptible de reconnaître les anticorps anti-protéine p24

Dans un flacon, on a préparé une solution de polymère AMVE-67 anhydride maléique vinyl éther (67 000 g/mol) à 1 g/l dans un mélange de DMSO (diméthylsulfoxyde)/eau 90/10 (V/V). On a laissé incubé le mélange pendant 48 h à 37°C. On a ensuite mélangé 36 µg de protéine p24 recombinante (pmR K24H, bioMérieux, Marcy l'Etoile, France) à 100 µl de la solution de polymère. On a laissé incubé 3 h à 37°C.

### 1.2 Préparation du partenaire de capture susceptible de reconnaître la cible ADN amplifiée à partir de l'ARN du virus VIH

On a dilué chacun des deux oligonucléotides suivants, C+ capable d'hybridation avec la cible ADN et C- incapable d'une telle hybridation, à une concentration de 10 µM dans un tampon PBS×3 (chlorure de sodium 0,45M, phosphate de sodium 0,15M, pH6,8)-EDTA 10mM.
C+ : NH₂-CGC TTC GAC AGC GAC GTG GGG
C- : NH₂-TAT GAA ACT TAT GGG GAT AC

### 1.3 Préparation des surfaces de capture

On a déposé sans contact, selon le procédé décrit dans la demande de brevet FR00/14691 tel que rappelé précédemment, 4 spots dans chaque puits d'une plaque de microtitration (Nunc Maxisorb). Deux des spots étaient constitués des conjugués protéine p24-polymère AMVE, un des spots était constitué de la sonde C+ et un de la sonde C-.

Après dépôt des spots, on a immédiatement placé la plaque dans une enceinte à 4°C pendant 2 h, puis on a laissé incuber pendant 30 min à 60°C.

### Exemple 2 : Préparation et capture spécifigue des analytes cible

### 2.1 Préparation des analytes cible

On a produit les cibles ADN (200 pdb) par RT-PCR d'une partie d'un gène viral extrait de culture cellulaire. On les a biotinylées par utilisation des amorces A et B suivantes marquées par la biotine en 5' :
Amorce A : CAT gTg CTA CTT CAC CAA Cgg
Amorce B : CTg gTA gTT gTg TCT gCA CAA

On a dénaturé ces cibles juste avant leur introduction par incubation avec le même volume de soude 0,2N pendant 5 min à température ambiante.

On a dilué différents sérums humains, pour lesquels on a préalablement testé la présence ou l'absence d'anticorps anti-protéine p24 par des méthodes classiques, au 1/500^{ème} dans un tampon de réaction A, de pH 7, constitué de : phosphate de sodium 0,1M, chlorure de sodium 0,5M, tween 20 0,65%, ADN de saumon 0,014% et PEG 4000 2%

### 2.2 Capture spécifique des analytes cibles

On a mélangé 30 µl de sérum de patient dilué, 30 µl de tampon de tampon de réaction A et 3 µl de cible ADN dénaturé et on déposé ce mélange dans chaque puits. On a laissé réagir 1 h à 37°C. On a ensuite rincé les puits par un tampon phosphate de sodium 50mM, chlorure de sodium 0,15M, tween 20 0,05% (PBS-tween).

### Exemple 3 : Détection des réactions d'hybridation et immunologiques

Dans chaque puits, on a déposé le mélange suivant: tampon de réaction A 30 µl, 10 ng d'un anticorps chèvre anti-humain conjugué à la péroxydase (Jackson ImmunoResearch), 0.07 pmole d'un oligonucléotide complémentaire de la cible ADN permettant ainsi de prendre celle-ci en sandwich entre la sonde de capture immobilisée en fond de plaque et la sonde de détection. Cet oligonucléotide a été préalablement marqué en 5' par la péroxydase. On a laissé réagir 1 h à 37°C. On a ensuite rincé les puits avec du PBS-tween.

On a ensuite ajouté dans chaque puits 30 µl d'un substrat précipitant colorimétrique de la péroxydase (TMB, bioFX). La révélation a lieu durant 20 minutes, puis on a éliminé le substrat par pipetage et on a mesuré la densité optique de chacun des spots à l'aide d'un microscope (Zeiss Axioplan2) connecté à une caméra CCD (Spot) et équipé d'un logiciel de traitement d'image (Image Pro+, Soft Imaging). Le logiciel permet de traduire l'intensité du signal de chaque spot en une valeur numérique comprise entre 0 (noir, pas de signal) et 255 (blanc, signal saturant).

A titre de comparaison, on a répété les modes opératoires décrits précédemment, mais on a omis la cible ADN afin de tester la spécificité d'hybridation ou bien on a utilisé des sérums dénués du virus (HIV-).

Les résultats obtenus sont indiqués dans le tableau 1 ci-dessous.

**Tableau 1**

| **Sérum** | **Présence de la cible** | **Intensité du spot** | **Intensité du spot** | **Intensité du spot** |
|---|---|---|---|---|
| | **ADN** | C+ | C- | **P24-AMVE** |
| HIV+ | Oui | 103 | 48 | 115 |
| HIV- | Oui | 108 | 45 | 45 |
| HIV+ | Non | 47 | 45 | 109 |
| HIV- | Non | 46 | 51 | 48 |

Les résultats ci-dessus montrent que la spécificité de la réaction est tout à fait satisfaisante. La présence de cibles ADN ne gêne pas la réaction de reconnaissance immunologique entre antigène P24 et anticorps anti-P24. A contrario, l'hybridation des cibles ADN sur les spots de sondes nucléiques est possible en présence d'anticorps dans le milieu. La présence de l'un ou l'autre des analytes ne génère pas non plus de bruit de fond indésirable. Le spot C- n'est jamais détecté, de même que les sérums HIV-.

### Exemple 4 : Détermination quantitative et qualitative de la détection selon le procédé de l'invention

On a répété le mode opératoire décrit dans les exemples 1 et 2, à ceci près qu'on a fait varier la concentration en cible ADN et la dilution du sérum VIH+.

La détection des réactions d'hybridation et immunologiques a ensuite été effectuée comme suit :
Dans chaque puits, on a déposé le mélange suivant: tampon de réaction A 30 µl, 10 ng d'un anticorps chèvre anti-humain conjugué à la phosphatase alcaline (Jackson ImmunoResearch), et 25 ng de streptavidine marquée par la phosphatase alcaline (Sigma). On a laissé réagir 1 h à 37°C. On a ensuite rincé les puits avec du PBS-tween.
On a ensuite ajouté dans chaque puits 30 µl d'un substrat fluorescent de la phosphatase alcaline (ECF, Amersham prêt à l'emploi). La révélation a lieu durant 5 min, puis on a éliminé le substrat par pipetage et on a mesuré l'intensité de fluorescence de chacun des spots comme indiqué dans l'exemple 3 ci-dessus..
Les résultats montrant le niveau de fluorescence par rapport à la dilution du sérum ou du produit PCR, sont représentés sur les figures 1A et 1B. Pour des raisons de clarté, on a reporté sur la figure 1A les signaux mesurés sur les spots des conjugués protéine p24-polymère AMVE et sur la figure 1B ceux mesurés sur les spots C+ (losanges) et C- (carrés), dans le même puits.
On observe sur la figure 1A une augmentation linéaire du signal de fluorescence en fonction de la concentration en sérum dans le puits, pour atteindre un plateau pour des dilutions inférieures au 1/1 000. Il est ainsi possible de réaliser des dosages quantitatifs par cette invention. La limite de détection est estimée selon une méthode statistique à 0.00001 ce qui correspond à une dilution du sérum au 1/100 000. Cette sensibilité élevée permet une détection précoce de la séroconversion suite à une infection HIV.

On obtient sur la figure 1B un profil similaire à celui observé avec les dilutions en cascade du sérum. Le signal de fluorescence augmente en fonction de la concentration en cible ADN sur le spot spécifique C+ (losanges). En revanche, le signal reste stable, proche de son niveau mesuré en l'absence de cible, sur le spot C- non hybridable avec la cible (carrés). La limite de détection de détection est estimée à 0,00007 soit une dilution du produit PCR au 1/14 000.

### Exemple 5 : Influence de la modification de la température et du tampon de réaction

On a répété le mode opératoire de l'exemple 4, à ceci près qu'on a également utilisé le tampon de réaction B, de pH 7,5, constitué de : HEPES 160mM, chlorure de lithium 0,5M, tween 20 0,05%, et qu'on a modifié la température pour qu'elle soit égale à 41 °C.

Dans cette expérience, le sérum contenait le virus (VIH+) et la cible ne reconnaissant pas l'ADN viral (C-) était absente.

Les résultats sont indiqués dans le tableau 2 ci-dessous.

**Tableau 2**

| **Température** | **Tampon de réaction** | **Signal du spot C+** | **Signal du spot P24-AMVE** |
|---|---|---|---|
| 37°C | A | 2,1 | 3,8 |
| 37°C | B | 1,8 | 2,7 |
| 41°C | A | 1,6 | 3,6 |
| 41°C | B | 1,3 | 1,8 |

### Exemple 6 : Détection simultanée des infections virales provoquées par les virus VIH, HBV et HCV dans une puce de détection

### 6.1 Outils biologiques pour le dosage de paramètres nucléiques et protéiques associés

### 6.1.1 Réactions d'hybridation

On a utilisé des cibles ARN HIV obtenues chez Ambion (Austin, Texas, USA). Les amorces biotinylées ((SK431 TGCTATGTCAGTTCCCCTTGGTTCTCT et SK462 AGTTGGAGGACATCAAGCAGCCATGCAAAT) et les sondes de capture, aminées, pour la capture des produits PCR (C_{HIV}: GAGACCATCAATGAGGAAGCTGCAGAATGGGAT) ont été synthétisées par Eurogentec (Seraing, Belgium).

Les ARN des virus HCV ont été extraits à partir de sérums de patients chroniques à l'aide du kit Nucleospin RNA Virus Kit (Macherey-Nagel, Hoerdt, France) et amplifiés par RT-PCR avec des amorces biotinylées (RC21: CTCCCGGGGCACTCGCAAGC et RC1:
GTGTAGCCATGGCGTTAGTA) (Roque Afonso A.M., 2000, Journal of Virological Methods,86, 55-60). La séquence de la sonde de capture HCV aminée est C_{HCV} (CATAGTGGTCTGCGGAACCGGTGAGT). Les ARN HIV et HCV ont été amplifiés par RT-PCR dans les conditions suivantes à l'aide du kit Access de Promega (Madison, WI, USA) : 1x AMV/Tfl tampon de réaction, 1,8 mM MgSO₄, 0,2 mM dNTP, 1 µM d'amorces, 1USI de AMV reverse transcriptase et 5USI de Tfl DNA polymerase; cycle RT 48°C, 45 min; 35 cycles PCR (94°C, 30 s ; 60°C,1 min ; 68°C, 2 min) ; extension finale à 68°C pendant 7 min.

Les amplicons ont été analysés sur gel d'agarose+bromure d'éthidium. Les concentrations des produits amplifiés ont été évaluées à l'aide d'un mass ladder (Eurogentec) (46 nM pour les amplicons HIV, 23 nM pour les amplicons HCV).

Une cible synthétique simple brin issue du gène HBV (74 pb) (CCCAGTAAAGTTCCCCACCTTATGAGTCCAAGGAATTACTAACATTGAGATTCCC GAGATTGAGATCTTCTGCGA), une sonde de capture aminée (C_{HBV}: ATCTCGGGAATCTCAATGTTAG) et une sonde de détection biotinylée (D_{HBV}: TATTCCGACTCATAAGGTG), toutes deux complémentaires de la cible HBV, ont été synthétisées chez Eurogentec.

### 6.1.2 Réactions immunologiques

La protéine core de HCV (HCV core) ainsi que la protéine d'enveloppe du VIH (gp160) ont été produites par la Demanderesse. On a utilisé deux antigènes de surface du VHB différents (Ag HBs), à savoir un antigène de sous-type Ay (Hytest, Turku, Finlande) et un antigène de sous-type Ad plasmatique Cliniqa (Fallbrook, CA, USA).

On a utilisé deux anticorps de souris non spécifiques de cette étude, à savoir un anticorps anti-TSH (NSP₁) et un anticorps anti-HCG (NSP₂), pour s'assurer de la spécificité des dosages.

Les sérums humains HIV, HBV et HCV ont été fournis par les hôpitaux lyonnais.

Les conjugués IgG de chèvre anti-humain marqués à la phosphatase alkaline ont été fournis par Jakson Immunoresearch (Wezst Grove, PA, USA) et la streptavidine marquée par la phosphatase alkaline provient de Sigma (St Quentin, France).

### 6.2 Préparation de la puce de détection et protocole des dosages biologiques

### 6.2.1 Dépôt des partenaires de capture

On a dilué les oligonucléotides à 10 µM dans un tampon de réaction d'adsorption des oligonucléotides (150 mM phosphate, 450 mM NaCl, 1 mM EDTA; pH7.4).

On a dilué les IgG non spécifiques à 50 µg/ml dans un tampon carbonate 50 mM pH9,3.

On a dilué les protéines gp160, Ag HBs et HCV core à 10 µg/ml dans du PBS.

On a réalisé les dépôts à l'aide de l'appareil Biochip Arrayer (Perkin Elmer, Boston, USA). On a déposé sous forme de spot, en duplica, sur un format circulaire de 16+1 spots dans des plaques de microtitration blanches (Greiner, Longwood, USA) (figure 2).

Après dépôt et incubation dans des conditions contrôlées de température (10°C) et d'humidité (50%), on a lavé (PBS tween 0,05%), séché et stocké à 4°C les plaques.

### 6.2.2 Capture des cibles nucléiques et des anticorps

On a utilisé les différentes combinaisons d'analytes suivantes :
I : cibles HBV,
II : cibles HIV
III : sérum HBV
IV : sérum HIV
V : cibles HBV+HIV
VI : cibles HBV+HIV+HCV
VII : cibles HBV+HIV+HCV + sérum HBV
VIII : cibles HBV+HIV+HCV + sérum HBV + sérum HIV

Pour ce faire, on a introduit dans chaque puits: 1,5 µl de produit d'amplification HIV ou HCV préalablement dénaturé par 1,5 µl de soude 0.2N ; 3 µl d'ADN synthétique HBV à 10 nM ; 1 µl de sérum humain dilué au 1/10. On a complété le volume à 30 µl avec le tampon de réaction (0.1M Na₂HPO₄/NaH₂PO₄; 0.5M NaCl; 0.65% tween 20; 2% PEG 4000; pH 7) et on a incubé les solutions durant 1h à 37°C, puis on les a rincées par un mélange PBS-tween 0.05%.

### 6.2.3 Détection

On a incubé les plaques avec une solution 0,2 µM de D_{HBV} (Détection HBV) pendant 30 minutes à 37°C, puis on les a rincées. On les a ensuite incubées en présence d'une solution de streptavidine-phosphatase alkaline (0,5 µg/ml) et d'anticorps de chèvre anti-humain marqués par la phosphatase alkaline ( µg/ml). On a ensuite lavé les plaques avec du PBS tween 0,05%, puis on a ajouté dans chaque puits un substrat précipitant de la phosphatase alkaline (BM purple BM, Roche, Basel, Suisse). On a photographié les plaques à l'aide d'une caméra CCD couplée à un logiciel d'analyse d'image qui permet de déterminer le niveau de gris moyen associé à chaque spot.

Les résultats sont indiqués dans le tableau 3 ci-dessous.

**Tableau 3**

| **Combinaison d'analytes** | **Position attendue des spots** | **Position obtenue des spots** | **Niveau de gris associé à chacun des analytes (a.u.)** |
|---|---|---|---|
| I | 3, 11 | 3, 11 | 34 935 |
| II | 1,9,17 | 1, 9, 17 | 38 144 |
| III | 8, 16 | 8, 16 | 33 664 |
| IV | 5, 13 | 5, 13 | 12 480 |
| V | 3, 11 | 3, 11 | 43 605 |
| | 1,9,17 | 1, 9, 17 | 49 470 |
| VI | 3, 11 | 3, 11 | 43 350 |
| | 1, 9, 17 | 1,9,17 | 48 832 |
| | 7, 15 | 7, 15 | 14917 |
| VII | 3, 11 | 3, 11 | 36 465 |
| | 1, 9, 17 | 1,9,17 | 42 712 |
| | 7, 15 | 7,15 | 11 857 |
| | 8, 16 | 8, 16 | 45 772 |
| VIII | 3, 11 | 3, 11 | 34 807 |
| | 1, 9, 17 | 1, 9, 17 | 40 290 |
| | 7, 15 | 7,15 | 11 730 |
| | 8, 16 | 8, 16 | 42 840 |
| | 5, 13 | 5, 13 | 12 240 |

Les spots pertinents apparaissent en fonction de la nature de l'analyte incubé, ce qui montre que les réactions d'hybridations et immunologiques ont lieu dans les mêmes conditions opératoires, notamment dans le même tampon et à la même température, même lorsque le partenaires de capture sont des marqueurs de maladies différentes.

## Revendications

1. Procédé de détection simultanée de réactions d'hybridation et immunologiques dans un échantillon susceptible de contenir des analytes cibles constitués d'au moins un acide nucléique et d'au moins un autre ligand de nature différente, comprenant les étapes consistant à :
(i) déposer une quantité connue en volume de l'échantillon dilué dans un tampon de réaction, sur une surface de capture préalablement revêtue des partenaires de capture desdits analytes cibles, lesdits partenaires de capture consistant en au moins une sonde nucléique et au moins un anti-ligand,
(ii) mettre à réagir à une température comprise entre 15°C et 60°C et
(iii) visualiser les réactions d'hybridation et immunologiques ainsi obtenues,
**caractérisé en ce que** le tampon de réaction a une force ionique comprise entre 0,4 et 1 M, a un pH compris entre 7 et 8 et contient un agent tensioactif.

2. Procédé selon la revendication 1, **caractérisé en ce qu'**il comprend l'étape supplémentaire suivante (i'), placée entre l'étape (i) et l'étape (ii), consistant à :
(i') ajouter des partenaires de liaison spécifiques desdits analytes cible, lesquels partenaires ont été préalablement conjugués à des marqueurs.

3. Procédé selon la revendication 1, **caractérisé en ce qu'**il comprend les deux étapes supplémentaires suivantes (ii') et (ii"), placées entre l'étape (ii) et l'étape (iii), consistant à :
(ii') ajouter des partenaires de liaison spécifiques desdits analytes cible, lesquels partenaires ont été préalablement conjugués à des marqueurs, et
(ii'') mettre à réagir à une température comprise entre 15°C et 60°C.

4. Procédé selon l'une des revendications 2 ou 3, **caractérisé en ce que** le ou les acide(s) nucléique(s) cible(s) contenu(s) dans l'échantillon biologique a(ont) été préalablement marqué(s) de sorte que les seuls partenaires de liaison à ajouter dans l'étape (i') ou (ii') sont le ou les partenaire(s) spécifique(s) du ligand, le(s)quel(s) a(ont) été préalablement marqué(s).

5. Procédé selon l'une quelconque des revendications 2 à 4, **caractérisé en ce que**, dans l'étape (i') ou (ii'), on ajoute, à la place du ou des partenaire(s) de liaison spécifique(s) de ligand, le ou les ligands susceptibles d'être contenu(s) dans l'échantillon biologique, le(s)quel(s) ligand(s) a(ont) été préalablement marqué(s).

6. Procédé selon l'une quelconque des revendications 2 à 5, **caractérisé en ce qu'**on ajoute le ou les substrat(s) spécifiques du ou des marqueur(s) avant l'étape (iii) de visualisation des réactions d'hybridation et immunologiques.

7. Procédé selon l'une quelconque des revendications 1 à 6, **caractérisé en ce que** la surface de capture est rincée après les étapes (ii) et/ou (ii").

8. Procédé selon l'une quelconque des revendications 1 à 7, **caractérisé en ce que** la température de réaction est comprise entre 35 et 45°C.

9. Procédé selon la revendication 8, **caractérisé en ce que** la température de réaction est comprise entre 37 et 41°C.

10. Procédé selon la revendication 9, **caractérisé en ce que** la température de réaction est de 37°C ou 41°C.

11. Procédé selon l'une quelconque des revendications 2 à 10, **caractérisé en ce que** le(s) partenaire(s) de liaison spécifique(s) d'acide nucléique et le(s) partenaire(s) de liaison spécifique(s) de ligand, le ou les acides nucléiques cibles tels que définis dans la revendication 4 et le ou les ligands tels que définis dans la revendication 5 sont marqués avec le même marqueur.

12. Procédé selon l'une quelconque des revendications 1 à 11, **caractérisé en ce que** les partenaires de capture sont des marqueurs d'une même maladie.

13. Procédé selon l'une quelconque des revendications 1 à 11, **caractérisé en ce que** les partenaires de capture sont des marqueurs de maladies différentes.

14. Utilisation du procédé selon l'une quelconque des revendications 1 à 13 pour la détection de maladies d'origine infectieuse ou métaboliques.

15. Utilisation du procédé selon la revendication 14, pour la détection de maladies d'origine virale.

16. Utilisation du procédé selon l'une quelconque des revendications 1 à 13 dans le diagnostic industriel de la présence de bactéries.

17. Utilisation du procédé selon l'une quelconque des revendications 1 à 13 pour l'identification et/ou la quantification de molécules biologiques.

18. Utilisation du procédé selon l'une quelconque des revendications 1 à 13 pour la détection simultanée du transcriptome et du protéome d'une cellule ou d'un organisme.

19. Kit de diagnostic comprenant une surface de capture, des partenaires de capture des analytes cibles consistant en au moins une sonde nucléique et au moins un autre antiligand de nature différente, et un tampon de réaction ayant une force ionique comprise entre 0,4 et 1 M, un pH compris entre 7 et 8 et contenant un agent tensioactif.

20. Kit de diagnostic selon la revendication 21, **caractérisé en ce qu'**il comprend également des partenaires de liaison spécifiques des ligands cibles préalablement marqués.

21. Kit de diagnostic selon la revendication 19, **caractérisé en ce qu'**il comprend également des ligands préalablement marqués susceptibles d'être contenus dans l'échantillon à tester, et un tampon de réaction.

22. Kit de diagnostic selon l'une des revendications 20 ou 21, **caractérisé en ce qu'**il comprend également des partenaires de liaison spécifiques des acides nucléiques cibles.

## Claims

1. A method for simultaneously detecting hybridization reactions and immunoreactions in a sample which may contain target analytes consisting of at least one nucleic acid and of at least one other ligand that is different in nature, **characterized in that** it comprises the steps consisting in:
(i) depositing a known amount of volume of the sample diluted in a reaction buffer, onto a capture surface pre-coated with the partners for capturing said target analytes, said capture partners consisting of at least one nucleic acid probe and at least one antiligand,
(ii) reacting at a temperature of between 15°C and 60°C and
(iii) visualizing the hybridization reactions and immunoreactions thus obtained,
**characterized in that** the reaction buffer has an ionic strength of between 0.4 and 1 M, has a pH of between 7 and 8, and contains a surfactant.

2. The method as claimed in claim 1, **characterized in that** it comprises the following additional step (i'), placed between step (i) and step (ii), consisting in:
(i') adding binding partners specific for said target analytes, which partners have been conjugated to labels beforehand.

3. The method as claimed in claim 1, **characterized in that** it comprises the following two additional steps (ii') and (ii"), placed between step (ii) and step (iii), consisting in:
(ii') adding binding partners specific for said target analytes, which partners have been conjugated to labels beforehand, and
(ii") reacting at a temperature of between 15°C and 60°C.

4. The method as claimed in either of claims 2 and 3, **characterized in that** the target nucleic acid(s) contained in the biological sample has(have) been prelabeled such that the only binding partners to be added in step (i') or (ii') are the partner(s) specific for the ligand, which partner(s) has(have) been prelabeled.

5. The method as claimed in any one of claims 2 to 4, **characterized in that** in step (i') or (ii'), the ligand(s) which may be contained in the biological sample, which ligand(s) has(have) been prelabeled, is(are) added in place of the ligand-specific binding partner(s).

6. The method as claimed in any one of claims 2 to 5, **characterized in that** the substrate(s) specific for the label(s) is(are) added before step (iii) for visualizing the hybridization reactions and immunoreactions.

7. The method as claimed in any one of claims 1 to 6, **characterized in that** the capture surface is rinsed after steps (ii) and/or (ii").

8. The method as claimed in any one of claims 1 to 7, **characterized in that** the reaction temperature is between 35 and 45°C.

9. The method as claimed in claim 8, **characterized in that** the reaction temperature is between 37 and 41°C.

10. The method as claimed in claim 9, **characterized in that** the reaction temperature is 37°C or 41 °C.

11. The method as claimed in any one of claims 2 to 10, **characterized in that** the nucleic acid-specific binding partner(s) and the ligand-specific binding partner(s), the target nucleic acid(s) as defined in claim 4 and the ligand(s) as defined in claim 5 are labeled with the same label.

12. The method as claimed in any one of claims 1 to 11, **characterized in that** the capture partners are markers for the same disease.

13. The method as claimed in any one of claims 1 to 11, **characterized in that** the capture partners are markers for different diseases.

14. The use of the method as claimed in any one of claims 1 to 13, for detecting diseases of infectious or metabolic origin.

15. The use of the method as claimed in claim 14, for detecting diseases of viral origin.

16. The use of the method as claimed in any one of claims 1 to 13, in the industrial diagnosis of the presence of bacteria.

17. The use of the method as claimed in any one of claims 1 to 13, for identifying and/or quantifying biological molecules.

18. The use of the method as claimed in any one of claims 1 to 13, for simultaneously detecting the transcriptome and the proteome of a cell or of an organism.

19. A diagnostic kit comprising a capture surface, partners for capturing the target analytes consisting of at least one nucleic acid probe and at least one other antiligand that is different in nature, and a reaction buffer having an ionic strength of between 0.4 and 1 M, a pH of between 7 and 8, and containing a surfactant.

20. The diagnostic kit as claimed in claim 19, **characterized in that** it also comprises binding partners specific for the target ligands, that are prelabeled.

21. The diagnostic kit as claimed in claim 19, **characterized in that** it also comprises prelabeled ligands which may be contained in the sample to be tested, and a reaction buffer.

22. The diagnostic kit as claimed in either of claims 20 and 21, **characterized in that** it also comprises binding partners specific for the target nucleic acids.

## Patentansprüche

1. Verfahren zum gleichzeitigen Nachweis von Hybridisierungs- und immunologischen Reaktionen in einer Probe, die Zielanalyten enthalten kann, die aus mindestens einer Nukleinsäure und mindestens einem anderen Liganden einer anderen Art bestehen, bei dem man in den folgenden Schritten:
(i) eine bekannte Menge an Probenvolumen, verdünnt in einem Reaktionspuffer, auf eine Einfangoberfläche aufträgt, die zuvor mit Einfangpartnern für die Zielanalyten beschichtet wurde, wobei die Einfangpartner aus mindestens einer Nukleinsäuresonde und mindestens einem Anti-Liganden bestehen,
(ii) bei einer Temperatur zwischen 15°C und 60°C reagieren lässt und
(iii) die so erhaltenen Hybridisierungs- und immunologischen Reaktionen sichtbar macht, **dadurch gekennzeichnet, dass** der Reaktionspuffer eine Ionenstärke zwischen 0,4 und 1 M und einen pH zwischen 7 und 8 aufweist und ein oberflächenaktives Mittel enthält.

2. Verfahren nach Anspruch 1, **dadurch gekennzeichnet, dass** es den folgenden zusätzlichen Schritt (i') zwischen dem Schritt (i) und dem Schritt (ii) enthält, wobei man:
(i') spezifische Bindungspartner der Zielanalyten zugibt, wobei die Partner zuvor mit Markern konjugiert wurden.

3. Verfahren nach Anspruch 1, **dadurch gekennzeichnet, dass** es die folgenden zwei zusätzlichen Schritte (ii') und (ii") zwischen dem Schritt (ii) und dem Schritt (iii) enthält, wobei man:
(ii') spezifische Bindungspartner der Zielanalyten zugibt, wobei die Partner zuvor mit Markern konjugiert wurden, und
(ii'') bei einer Temperatur zwischen 15°C und 60°C reagieren lässt.

4. Verfahren nach einem der Ansprüche 2 oder 3, **dadurch gekennzeichnet, dass** die in der biologischen Probe enthaltene(n) Ziel-Nukleinsäure(n) zuvor markiert wurde(n), so dass die einzigen Bindungspartner, die im Schritt (i') oder (ii') zugegeben werden müssen, der oder die spezifische(n) Bindungspartner des Liganden ist/sind, der (die) zuvor markiert wurde(n).

5. Verfahren nach einem der Ansprüche 2 bis 4, **dadurch gekennzeichnet, dass** man im Schritt (i') oder (ii') anstelle des oder der spezifische(n) Liganden-Bindungspartner(s) den oder die Liganden hinzufügt, der (die) in der biologischen Probe enthalten sein kann (können), wobei der (die) Ligand(en) zuvor markiert wurde(n).

6. Verfahren nach einem der Ansprüche 2 bis 5, **dadurch gekennzeichnet, dass** man das oder die spezifische(n) Substrat(e) des oder der Marker vor dem Schritt (iii) hinzufügt, in dem die Hybridisierungs-und immunologischen Reaktionen sichtbar gemacht werden.

7. Verfahren nach einem der Ansprüche 1 bis 6, **dadurch gekennzeichnet, dass** die Einfangoberfläche nach den Schritten (ii) und/oder (ii'') gespült wird.

8. Verfahren nach einem der Ansprüche 1 bis 7, **dadurch gekennzeichnet, dass** die Reaktionstemperatur zwischen 35 und 45°C liegt.

9. Verfahren nach Anspruch 8, **dadurch gekennzeichnet, dass** die Reaktionstemperatur zwischen 37 und 41°C liegt.

10. Verfahren nach Anspruch 9, **dadurch gekennzeichnet, dass** die Reaktionstemperatur 37°C oder 41°C beträgt.

11. Verfahren nach einem der Ansprüche 2 bis 10, **dadurch gekennzeichnet, dass** der (die) spezifische(n) Nukleinsäure-Bindungspartner und der (die) spezifische(n) Liganden-Bindungspartner, die Zielnukleinsäure(n) nach Anspruch 4 und der oder die Ligand(en) nach Anspruch 5 mit demselben Marker markiert sind.

12. Verfahren nach einem der Ansprüche 1 bis 11, **dadurch gekennzeichnet, dass** die Einfangpartner Marker für dieselbe Krankheit sind.

13. Verfahren nach einem der Ansprüche 1 bis 11, **dadurch gekennzeichnet, dass** die Einfangpartner Marker für unterschiedliche Krankheiten sind.

14. Verwendung des Verfahrens nach einem der Ansprüche 1 bis 13 zum Nachweis von Krankheiten infektiösen oder metabolischen Ursprungs.

15. Verwendung des Verfahrens nach Anspruch 14 zum Nachweis von Krankheiten viralen Ursprungs.

16. Verwendung des Verfahrens nach einem der Ansprüche 1 bis 13 zur industriellen Diagnostik des Vorliegens von Bakterien.

17. Verwendung des Verfahrens nach einem der Ansprüche 1 bis 13 zur Identifikation und/oder zur Quantifizierung biologischer Moleküle.

18. Verwendung des Verfahrens nach einem der Ansprüche 1 bis 13 zum gleichzeitigen Nachweis des Transkriptoms und des Proteoms einer Zelle oder eines Organismus.

19. Diagnostisches Kit, umfassend eine Einfangoberfläche, Einfangpartner für die Zielanalyten, die aus mindestens einer Nukleinsäuresonde und mindestens einem anderen Anti-Liganden anderer Art bestehen, und einen Reaktionspuffer mit einer Ionenstärke zwischen 0,4 und 1 M und einem pH zwischen 7 und 8, der ein oberflächenaktives Mittel enthält.

20. Diagnostisches Kit nach Anspruch 21, **dadurch gekennzeichnet, dass** es außerdem zuvor markierte, spezifische Bindungspartner der Zielliganden umfasst.

21. Diagnostisches Kit nach Anspruch 19, **dadurch gekennzeichnet, dass** es außerdem zuvor markierte Liganden, die in der zu testenden Probe enthalten sein können, und einen Reaktionspuffer umfasst.

22. Diagnostisches Kit nach einem der Ansprüche 20 oder 21, **dadurch gekennzeichnet, dass** es außerdem spezifische Bindungspartner der Zielnukleinsäuren umfasst.


### SEQUENCE LISTING

<110> bioMérieux
<120> Procédé de détection simultanée de réactions d'hybridation et immunologiques et utilisations
<130> Multidétection
<150> FR 02/06883
   <151> 2002-06-05
<160> 13
<170> PatentIn version 3.1
<210> 1
   <211> 21
   <212> DNA
   <213> artificial sequence
<220>
   <223> oligonucléotide de capture C+
<400> 1
   cgcttcgaca gcgacgtggg g 21
<210> 2
   <211> 20
   <212> DNA
   <213> artificial sequence
<220>
   <223> Oligonucléotide de capture C-
<400> 2
   tatgaaactt atggggatac 20
<210> 3
   <211> 21
   <212> DNA
   <213> artificial sequence
<220>
   <223> Amorce A
<400> 3
   catgtgctac ttcaccaacg g 21
<210> 4
   <211> 21
   <212> DNA
   <213> artificial sequence
<220>
   <223> Amorce B
<400> 4
   ctggagttg tgtctgcaca a 21
<210> 5
   <211> 27
   <212> DNA
   <213> artificial sequence
<220>
   <223> amorce SK431
<400> 5
   tgctatgtca gttccccttg gttctct 27
<210> 6
   <211> 30
   <212> DNA
   <213> artificial sequence
<220>
   <223> amorce SK462
<400> 6
   agttggagga catcaagcag ccatgcaaat 30
<210> 7
   <211> 33
   <212> DNA
   <213> artificial sequence
<220>
   <223> sonde de capture C HIV
<400> 7
   gagaccatca atgaggaagc tgcagaatgg gat 33
<210> 8
   <211> 20
   <212> DNA
   <213> artificial sequence
<220>
   <223> amorce RC21
<400> 8
   ctcccggggc actcgaagc 20
<210> 9
   <211> 20
   <212> DNA
   <213> artificial sequence
<220>
   <223> amorce RC1
<400> 9
   gtgtagccat ggcgttagta 20
<210> 10
   <211> 26
   <212> DNA
   <213> artificial sequence
<220>
   <223> sonde de capture C HCV
<400> 10
   catagtggtc tgcggaaccg gtgagt 26
<210> 11
   <211> 75
   <212> DNA
   <213> artificial sequence
<220>
   <223> cible HBV
<400> 11
   cccagtaaag ttccccacct tatgagtcca aggaattact aacattgaga ttcccgagat 60
   tgagatcttc tgcga 75
<210> 12
   <211> 22
   <212> DNA
   <213> artificial sequence
<220>
   <223> sonde de capture HBV
<400> 12
   atctcgggaa tctcaatgtt ag 22
<210> 13
   <211> 19
   <212> DNA
   <213> Artificial sequence
<220>
   <223> D HBV
<400> 13
   tattccgact cataaggtg 19